# FASCICULE DE BREVET EUROPEEN

(11) **EP 1 040 822 B1**
(45) Date de publication et mention de la délivrance du brevet: **09.06.2004**
(21) Numéro de dépôt: 00400880.1
(22) Date de dépôt: 30.03.2000
(51) Int. Cl.: A61K 7/48, A61K 7/00

(54) **Masque extensible comportant une matrice adhésive étirable au moins à l'état mouillé.**
Dehnbare Maske mit einer Haftmatrix , die zumindest im feuchten Zustand gedehnt werden kann
Extensible Mask with an adhesive matrix which is at least stretchable when wetted

(30) Priorité: 01.04.1999 FR 9904094
(43) Date de publication de la demande: 04.10.2000
(73) Titulaire: L'OREAL, 75008 Paris (FR)
(72) Inventeur: Guerret, Jean-Louis, 75016 Paris (FR)
(74) Mandataire: Leszczynski, André

(56) Documents cités:
- EP-A- 0 309 309
- WO-A-96/14822
- WO-A-97/32567
- FR-A- 2 538 247
- DATABASE WPI Section Ch, Week 199412 Derwent Publications Ltd., London, GB; Class A96, AN 1994-097759 XP002125705 & JP 06 048917 A (NAKAGAWA M), 22 février 1994 (1994-02-22)

## Description

La présente invention concerne un masque pour le visage ou le corps.

Il existe un besoin pour disposer d'un masque qui puisse s'adapter aisément à la morphologie de l'utilisateur afin d'obtenir notamment un traitement plus efficace.

L'invention vise ainsi à proposer un nouveau masque capable de s'adapter aisément aux différents contours et reliefs du visage ou de la partie du corps sur laquelle il est appliqué.

Le masque selon l'invention comporte une feuille de support revêtue sur une face d'une matrice adhésive comprenant un adhésif permanent adhérent sur peau sèche et mouillée, l'un au moins de la feuille de support et de la matrice adhésive comprenant au moins un actif destiné à exercer une action spécifique sur la peau, et se caractérise par le fait que la matrice adhésive est étirable au moins à l'état mouillé afin de permettre une adaptation du masque à la forme du visage ou de la partie du corps à traiter, la feuille de support étant choisie pour pouvoir, au moins à l'état mouillé, s'étirer afin d'accompagner les déformations de la matrice.

Grâce à l'invention, on dispose d'un masque qui permet un traitement plus efficace que certains masques connus non étirables et n'adhérant que trop faiblement à la peau pour permettre un bon positionnement lors de la pose.

Dans une réalisation particulière, le masque présente au moment de l'utilisation, dans au moins une direction, un facteur d'élongation compris entre 5 et 30 %, de préférence entre 10 et 30 % et de préférence encore entre 15 et 30 %.

De préférence, l'adhésif est hydrophobe.

De préférence encore, la matrice adhésive forme une couche occlusive.

La feuille de support peut comporter des perforations et/ou des découpes destinées à la rendre extensible ou à augmenter son facteur d'élongation.

Ces perforations peuvent être en forme de losange, par exemple.

Les découpes peuvent se présenter sous la forme de fentes disposées en quinconce, par exemple.

Toujours dans une réalisation particulière, la matrice adhésive comporte une ou plusieurs substances hydroabsorbantes, par exemple des polyacrylates, des particules ou fibres naturelles ou synthétiques hydroabsorbantes, des alginates, des dérivés de maïs ou de cellulose.

Dans une réalisation particulière, la matrice adhésive comporte un ou plusieurs actifs hydrosolubles et/ou liposolubles.

La matrice adhésive peut comporter du collagène.

La matrice adhésive et/ou la feuille de support peuvent comporter des extraits secs d'origine animale ou végétale et/ou des substances minérales.

Toujours dans une réalisation particulière, la feuille de support est réalisée dans un matériau hydrogonflable.

La feuille de support peut encore être constituée par un tissé élastique.

En variante, la feuille de support peut aussi être constituée par un non-tissé hydrophyle.

La feuille de support peut comporter une ou plusieurs substances ou particules ou fibres hydroabsorbantes, par exemple des polyacrylates et/ou une proportion importante de viscose ou de coton, par exemple entre 30 et 90 % (en masse).

La feuille de support peut comporter du polypropylène ou du polyester, par exemple entre 5 et 95 % (en masse).

Dans une réalisation particulière, le masque comporte des actifs choisis pour exercer une action choisie dans la liste non limitative suivante : hydrater, niveler, éclaircir, tonifier, amincir, cicatriser, insensibiliser.

Dans une réalisation particulière, l'adhésif et la feuille de support sont choisis de telle sorte qu'une fois mouillé, le masque présente une surface à l'état étiré supérieure de 5 à 70 % à la surface du masque à l'état sec, et de préférence supérieure de 10 à 70 % et de préférence encore de 20 à 70 %.

L'adhésif peut être choisi dans la liste suivante : adhésifs à base acrylique, vinylique, polyuréthane, silicone ou élastomère (butyle ou latex par exemple) ou des copolymères et/ou mélanges de ceux-ci.

Avantageusement, l'adhésif est choisi de manière à présenter, lorsqu'il est appliqué à l'état fluide sur la feuille de support lors de la fabrication du masque, une viscosité lui permettant de ne pas la traverser.

La matrice adhésive peut comporter des plastifiants, des huiles ou des polyols (glycérine par exemple) pour la rendre suffisamment étirable.

La feuille de support et/ou la matrice adhésive peuvent être colorées.

Dans une réalisation particulière, la feuille de support n'est pas étirable tant qu'elle n'est pas imprégnée d'eau et de préférence elle n'est pas étirable non plus lorsqu'elle est au contact de la matrice à l'état fluide, en phase solvant, lors de la fabrication du masque.

L'invention a encore pour objet l'utilisation d'un masque tel que défini plus haut pour traiter le visage, le cou ou les cuisses par exemple, le masque étant trempé dans l'eau avant l'application, puis appliqué sur la peau de préférence préalablement mouillée en étant étiré de manière à s'adapter à la morphologie de l'utilisateur, ou en variante appliqué à l'état sec sur peau mouillée, la feuille de support étant ensuite aspergée d'eau.

L'invention sera mieux comprise à la lecture de la description détaillée qui va suivre, d'exemples de réalisation non limitatifs de l'invention, et à l'examen du dessin annexé sur lequel :
- la figure 1 est une vue en coupe, très schématique, illustrant la structure d'un masque conforme à un exemple de réalisation de l'invention,
- les figures 2 à 5 représentent différentes variantes de perforations ou de découpes de la feuille de support, destinées à favoriser sa déformation, et
- les figures 6 et 7 représentent respectivement le masque à l'état initial et à l'état étiré.

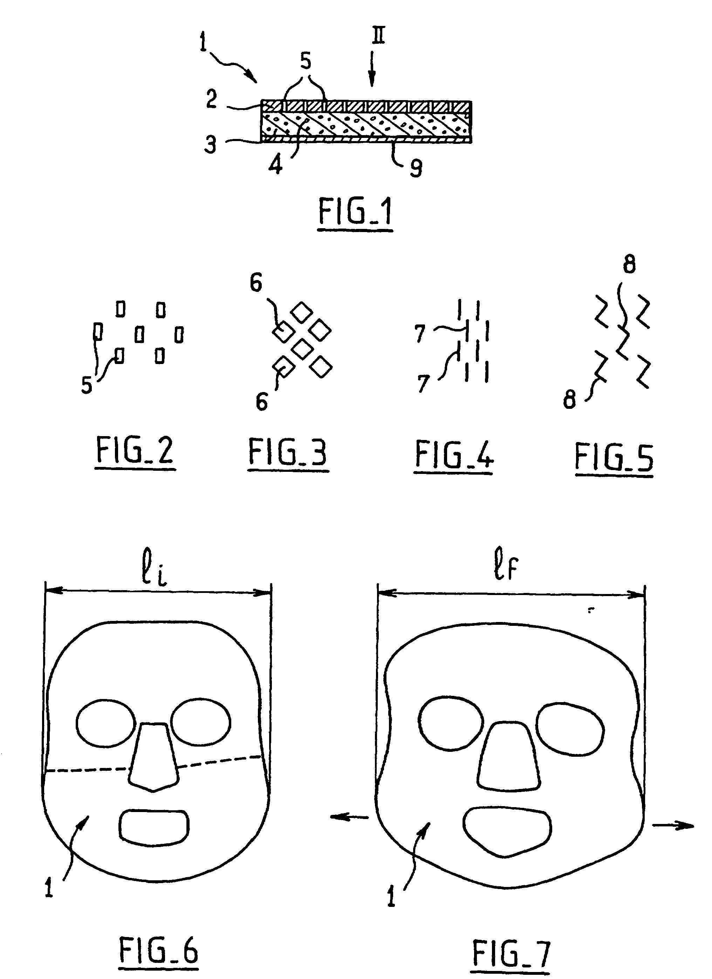

Dans l'exemple de réalisation représenté sur la figure 1, le masque comporte une feuille de support 2 revêtue sur une face d'une matrice adhésive 3 contenant un adhésif et des actifs 4 noyés dans l'adhésif.

Une pellicule de protection amovible 9 protège la matrice adhésive 3 avant l'utilisation.

La feuille de support 2 comporte des perforations 5 ayant une forme sensiblement rectangulaire lorsqu'observées de dessus, comme on peut le voir sur la figure 2.

La feuille de support 2 est constituée dans l'exemple décrit par un non tissé hydrogonflable de densité comprise entre 20 et 100 g/m², par exemple 50 g/m², comportant 30 % de fibres de polyester et 70 % de fibres de viscose.

L'adhésif est ici un adhésif permanent à base acrylique.

Parmi les autres adhésifs utilisables, on peut citer notamment les adhésifs à base vinylique (par exemple l'acétate de vinyle), polyuréthane, silicone ou élastomère (butyle ou latex par exemple), les adhésifs utilisés pouvant être partiellement réticulés.

Lors de la fabrication du masque, la matrice adhésive 3 est couchée à l'état fluide en phase solvant sur la feuille de support, sa viscosité étant suffisamment importante pour l'empêcher de traverser la feuille de support.

D'une manière générale, la matrice adhésive 3 est déposée en une quantité comprise de préférence entre 15 et 70 g/m², et présente de préférence un pouvoir adhésif (Tackiness Adhesive Coefficient) compris entre 200 et 600 g/cm² sur peau sèche et entre 20 et 150 g/cm² sur peau mouillée.

Dans l'exemple décrit, la matrice adhésive est déposée avec une quantité de 40g/m² et constitue une couche occlusive.

Le masque qui vient d'être décrit est non étirable à l'état sec, car la feuille de support 2 est non étirable à l'état sec.

Le masque est découpé à la forme de la partie du corps à traiter et comporte le cas échéant des découpes pour les yeux, le nez et la bouche. Eventuellement, le masque est en deux parties, la frontière étant matérialisée par un trait discontinu sur la figure 6.

Pour utiliser le masque, l'utilisateur le trempe dans l'eau puis l'applique sur la peau, après avoir mouillé celle-ci au préalable. En variante, le masque peut être appliqué à l'état sec sur la peau mouillée, puis la feuille de support est aspergée d'eau.

La feuille de support 2 chargée d'eau gonfle et s'étend, ce qui permet à la matrice adhésive 3 d'être étirée par l'utilisateur. Dans l'exemple décrit, le facteur d'élongation d'un rectangle de quelques cm² découpé dans le masque est d'environ 15 % dans le sens longitudinal.

L'utilisateur commence par positionner un bord du masque sur la peau puis, de proche en proche, l'ajuste sur la totalité de la surface à traiter.

Les propriétés de la matrice adhésive permettent de repositionner le masque.

Grâce à l'étirabilité de la feuille de support et de la matrice adhésive le masque est adapté au mieux à la morphologie de l'utilisateur.

La totalité de la surface de la matrice adhésive 3 peut ainsi venir au contact de l'ensemble du visage ou de la partie du corps à traiter.

On a représenté sur la figure 7, à titre d'exemple, la forme du masque 1 une fois étiré pour se conformer aux reliefs et contour du visage de l'utilisateur.

A titre indicatif, dans l'exemple décrit, la largeur initiale du masque est lᵢ = 15 cm environ à l'état sec et passe à l_{f} = 18 cm à l'état mouillé et étiré.

La feuille de support 2 peut être réalisée de diverses manières sans sortir du cadre de la présente invention.

Elle peut ne comporter aucune perforation, alvéole ou découpe mais être simplement réalisée dans un matériau hydrogonflable capable d'accompagner une fois imprégné d'eau les déformations de la matrice adhésive lorsque le masque est appliqué sur le visage.

La feuille de support peut également comporter tous types de perforations ou d'alvéoles permettant d'accroître son extensibilité, par exemple des perforations 6 en forme de losange comme illustré sur la figure 3.

La feuille de support 2 peut encore comporter des fentes disposées en quinconce, permettant au masque de s'étirer dans une direction perpendiculaire aux fentes, par écartement des bords des fentes, à la manière d'un métal déployé.

La feuille de support 2 peut aussi comporter des découpes 8 en forme de Z, disposées en quinconce, comme illustré sur la figure 5. Les bords de ces découpes peuvent s'écarter lorsque le masque est étiré.

Tous types d'actifs 4 peuvent être incorporés à la matrice adhésive 3 et/ou à la feuille de support 2, en fonction du traitement que l'on souhaite effectuer.

La matrice adhésive 3 peut notamment comprendre des particules capables d'absorber l'humidité, de manière à pomper l'eau dans la matrice adhésive 3 et favoriser la solubilisation d'actifs hydrophiles destinés à exercer un effet déterminé sur la peau.

Parmi les actifs utilisables, on peut citer des agents nettoyants anti-oxydants, anti-radicaux libres, hydratants, dépigmentants, liporégulateurs, anti-acnéiques, anti-séborrhiques, anti-vieillissement, adoucissants, anti-rides, kératolitiques, anti-inflammatoires, rafraîchissants, cicatrisants, protecteurs vasculaires, anti-bactériens, antifongiques, anti-perspirants, déodorants, conditionneurs de la peau, insensibilisants, immunomodulateurs, nourrissants, absorbeurs de sébum (Orgasol par exemple) ou d'humidité.

La matrice adhésive 3 peut ainsi comporter un actif hydrosoluble choisi parmi les composés suivants : l'acide ascorbique et ses sels biologiquement compatibles, les enzymes, des antibiotiques, les composants à effet tenseur, les α-hydroxy acides et leurs sels, les polyacides hydroxylés, les sucroses et leurs dérivés, l'urée, les aminoacides, les oligopeptides, les extraits végétaux hydrosolubles et de levures, les hydrolysats de protéines, l'acide hyaluronique, les mucopolysaccharides, les vitamines B₂, B₆, H, PP, le panthénol, l'acide folique, l'acide acétyl salicylique, l'allantoïne, l'acide glycyrrhétique, l'acide kojique, l'hydroquinone.

La matrice adhésive 3 peut encore comporter au moins un actif liposoluble choisi parmi les composés suivants : D-α-tocophérol, DL-α-tocophérol, acétate de D-α-tocophérol, acétate de DL-α-tocophérol, palmitate d'ascorbyle, vitamine F et glycérides de vitamine F, vitamine D, vitamine D₂, vitamine D₃, rétinol, esters de rétinol, palmitate de rétinol, propionate de rétinol, β-carotène, D-panthénol, farnesol, acétate de farnesyle ; huiles de jojoba et de cassis riches en acides gras essentiels ; les kératolytiques tels que l'acide salicylique, ses sels et ses esters, l'acide n-octanoyl-5 salicylique et ses esters, alkylesters d'α-hydroxyacides tels que l'acide citrique, acide lactique, acide glycolique ; acide asiatique, acide madécassique, asiaticoside, extrait total de centella asiatica, acide β-glycyrrhétinique, α-bisabolol, céramides comme le 2 oleoylamino-1,3 octadécane ; phytanetriol, sphingomycline de lait, phospholipides d'origine marine riches en acides gras essentiels polyinsaturés, éthoxyquine ; extrait de romarin, extrait de mélisse, quercetine, extrait de microalgues séchées, anti-inflammatoires stéroïdiens.

De tels actifs peuvent être incorporés à l'état solubilisé dans des huiles, utilisées seules ou en combinaison, parmi lesquelles on peut citer : les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de sésame, de noisette, de pistache, d'abricot, d'amandes ou d'avocat ; les huiles de poisson, le tricaproccaprylate de glycérol, ou les huiles végétales ou animales de formule R₁COOR₂ dans laquelle R₁ représente le reste d'un acide gras supérieur comportant de 7 à 19 atomes de carbone et R₂ représente une chaîne hydrocarbonée ramifiée contenant de 3 à 20 atomes de carbone, par exemple, l'huile de Purcellin ; l'huile de germes de blé, de calophyllum, de sésame, de coriandre, de carthame, l'huile de passiflore, l'huile de rosa mosqueta, de macadamia, de pépins de fruits (raisin, cassis, orange, kiwi), de colza, de coprah, d'arachide, d'onagre, de palme, de ricin, de lin, de jojoba, de chia, d'olive ou de germes de céréales comme l'huile de germes de blé, l'huile de son, de riz, l'huile de karité ; des acétylglycérides ; des octanoates, décanoates ou ricinoléates d'alcools ou de polyalcools ; des triglycérides d'acides gras ; des glycérides ; l'huile de paraffine, de vaseline, le perhydrosqualène ; des alcools gras (alcool stéarylique, alcool cétylique) et des acides gras (acide stéarique) et leurs esters ; les polyalkyl (C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA).

On peut encore citer les huiles hydrocarbonées partiellement fluorées ou les huiles perfluorées, et notamment les perfluoropolyéthers et les perfluoroalcanes.

La phase huileuse, c'est-à-dire les gouttelettes d'huile dispersées dans la couche de polymère, peut être présente dans une proportion allant de 0,1 % à 30 % en poids par rapport au poids total de la composition. De façon préférentielle, ce pourcentage est compris entre 5 et 25 %.

Alternativement, le ou les actifs liposolubles sont incorporés dans une couche de polymère hydrophobe, sous forme de poudre ou de granulés.

Pour appliquer la matrice adhésive 3 sur la feuille de support 2 lors de la fabrication, on fait défiler la feuille de support 2 et l'on dépose par enduction la matrice adhésive 3, alors en phase solvant.

Le solvant est ensuite évaporé.

Il est avantageux que la feuille de support 2 au contact du solvant précité ne soit pas étirable, au moins dans sa direction de défilement lors de l'enduction, car cela facilite son guidage et son bobinage.

L'invention n'est pas limitée aux exemples de réalisation décrits, et l'on peut par exemple combiner perforations et découpes au sein d'une même feuille de support.

## Revendications

1. Masque pour le visage ou le corps, comportant une feuille de support revêtue sur une face d'une matrice adhésive comprenant un adhésif permanent adhérant à la fois sur peau sèche et mouillée, l'un au moins de la feuille de support et de la matrice adhésive comprenant au moins un actif (4) destiné à exercer une action spécifique sur la peau, **caractérisé par le fait que** la matrice adhésive (3) est étirable au moins à l'état mouillé afin de permettre une adaptation du masque à la forme du visage ou de la partie du corps à traiter, la feuille de support (2) étant choisie pour pouvoir, au moins à l'état mouillé, s'étirer afin d'accompagner les déformations de la matrice adhésive (3).

2. Masque selon la revendication 1, **caractérisé par le fait qu'**il présente lors de l'utilisation, dans au moins une direction, un facteur d'élongation compris entre 5 et 30 %, de préférence entre 10 et 30 % et de préférence encore entre 15 et 30 %.

3. Masque selon la revendication 1 ou 2, **caractérisé par le fait que** l'adhésif est hydrophobe.

4. Masque selon l'une quelconque des revendications précédentes, **caractérisé par le fait que** la matrice adhésive (3) forme une couche occlusive.

5. Masque selon l'une quelconque des revendications précédentes, **caractérisé par le fait que** la feuille de support (2) comporte des perforations (5 ; 6) et/ou des découpes (7 ; 8).

6. Masque selon la revendication précédente, **caractérisé par le fait que** la feuille de support comporte des perforations (6) en forme de losange.

7. Masque selon la revendication 5, **caractérisé par le fait que** la feuille de support comporte des fentes (7) disposées en quinconce.

8. Masque selon l'une quelconque des revendications précédentes, **caractérisé par le fait que** la matrice adhésive (3) est choisie de manière à présenter, lorsqu'elle est appliquée à l'état fluide sur la feuille de support lors de la fabrication du masque, une viscosité lui permettant de ne pas la traverser.

9. Masque selon l'une quelconque des revendications précédentes, **caractérisé par le fait qu'**il comporte un ou plusieurs actifs (4) hydrosolubles et/ou liposolubles noyés dans la matrice adhésive (3).

10. Masque selon l'une quelconque des revendications précédentes, **caractérisé par le fait que** la matrice adhésive (3) est déposée en une quantité comprise entre 15 et 70 g/m2 sur la feuille de support (2).

11. Masque selon l'une quelconque des revendications précédentes, **caractérisé par le fait que** l'adhésif est choisi dans la liste suivante : adhésifs à base acrylique, vinylique, polyuréthane, silicone ou élastomère, les copolymères et/ou les mélanges de ceux-ci.

12. Masque selon l'une quelconque des revendications précédentes, **caractérisé par le fait qu'**il comporte une ou plusieurs substances hydroabsorbantes noyées dans la matrice adhésive (3), par exemple des polyacrylates ou alginates, des dérivés du maïs ou de la cellulose, des particules ou des fibres naturelles ou synthétiques hydroabsorbantes.

13. Masque selon l'une quelconque des revendications précédentes, **caractérisé par le fait que** la feuille de support (2) est réalisée dans un matériau hydrogonflable.

14. Masque selon l'une quelconque des revendications précédentes, **caractérisé par le fait que** la feuille de support (2) est constituée par un tissé élastique.

15. Masque selon l'une quelconque des revendications précédentes, **caractérisé par le fait que** la feuille de support est constituée par un non-tissé hydrophyle.

16. Masque selon l'une quelconque des revendications précédentes, **caractérisé par le fait que** la feuille de support comporte une ou plusieurs substances ou particules ou fibres hydroabsorbantes.

17. Masque selon l'une des deux revendications précédentes, **caractérisé par le fait que** le non-tissé comporte une proportion importante de viscose ou de coton, de préférence entre 30 et 90 % (en masse).

18. Masque selon l'une quelconque des revendications précédentes, **caractérisé par le fait que** la feuille de support comporte du polypropylène ou du polyester, de préférence dans une proportion comprise entre 5 et 95 % (en masse).

19. Masque selon l'une quelconque des revendications précédentes, **caractérisé par le fait que** l'adhésif et la feuille de support sont choisis de telle sorte qu'une fois mouillé, le masque présente une surface à l'état étiré supérieure de 5 à 70 % à la surface du masque à l'état sec, et de préférence supérieure de 10 à 70 % et de préférence encore supérieure de 20 à 70 %.

20. Masque selon l'une quelconque des revendications précédentes, **caractérisé par le fait qu'**il comporte des actifs (4) choisis pour exercer une action choisie dans la liste suivante : hydrater, niveler, éclaircir, tonifier, amincir, cicatriser, insensibiliser.

21. Masque selon l'une quelconque des revendications précédentes, **caractérisé par le fait que** la feuille de support est non étirable tant qu'elle n'est pas imprégnée d'eau et de préférence elle n'est pas étirable non plus lorsqu'elle est au contact de la matrice à l'état fluide, en phase solvant, lors de la fabrication du masque.

22. Utilisation d'un masque tel que défini dans l'une quelconque des revendications précédentes pour traiter le visage ou le cou.

23. Utilisation d'un masque tel que défini dans l'une quelconque des revendications 1 à 21 pour traiter les cuisses.

24. Utilisation du masque tel que défini dans l'une quelconque des revendications 1 à 21, le masque étant trempé dans l'eau avant l'application, puis appliqué sur la peau de préférence préalablement mouillée en étant étiré de manière à s'adapter à la morphologie de l'utilisateur.

25. Utilisation du masque tel que défini dans l'une quelconque des revendications 1 à 21, **caractérisé par le fait qu'**il est appliqué à l'état sec sur peau mouillée puis la feuille de support est aspergée d'eau.

## Patentansprüche

1. Gesichts- oder Körpermaske, umfassend einen Trägerbogen überzogen auf einer Oberfläche einer Klebmatrix, umfassend ein Adhesiv, permanent haftend sowohl auf trockener als auch auf angefeuchteter Haut, wobei mindestens eines von dem Trägerbogen und der Klebmatrix mindestens einen aktiven Bestandteil (4) enthält, bestimmt zum Ausüben einer spezifischen Wirkung auf die Haut, **dadurch gekennzeichnet, dass** die Klebmatrix (3) mindestens im angefeuchteten Zustand dehnbar ist, um eine Anpassung der Maske an die Form des Gesichts oder des zu behandelnden Körperteils zu gestatten, wobei der Trägerbogen (2) im Hinblick auf sein Vermögen gewählt ist, sich mindestens im angefeuchteten Zustand zu dehnen, um sich den Deformationen der Klebmatrix (3) anzupassen.

2. Maske gemäß Anspruch 1, **dadurch gekennzeichnet, dass** sie bei Anwendung in mindestens einer Richtung einen Dehnungsfaktor zwischen 5 und 30 %, vorzugsweise zwischen 10 und 30 % und noch stärker bevorzugt zwischen 15 und 30 % aufweist.

3. Maske gemäß Anspruch 1 oder 2, **dadurch gekennzeichnet, dass** das Adhesiv hydrophob ist.

4. Maske nach einem der vorstehenden Ansprüche, **dadurch gekennzeichnet, dass** die Klebmatrix (3) eine abschließende bzw. verschließende Schicht bildet.

5. Maske nach einem der vorstehenden Ansprüche, **dadurch gekennzeichnet, dass** der Trägerbogen (2) Perforationen (5, 6) und/oder Schnitte bzw. Stanzungen (7, 8) aufweist.

6. Maske nach dem vorstehenden Anspruch, **dadurch gekennzeichnet, dass** der Trägerbogen Perforationen (6) in Form von Rauten aufweist.

7. Maske gemäß Anspruch 5, **dadurch gekennzeichnet, dass** der Trägerbogen Schlitze (7) in Fünferanordnungen aufweist.

8. Maske nach einem der vorstehenden Ansprüche, **dadurch gekennzeichnet, dass** die Klebmatrix (3) so gewählt ist, dass sie dann, wenn sie in fluidem Zustand auf den Trägerbogen bei Herstellung der Maske aufgetragen wird, eine Viskosität aufweist, welche ihr nicht gestattet ihn zu durchdringen.

9. Maske nach einem der vorstehenden Ansprüche, **dadurch gekennzeichnet, dass** sie einen oder mehrere wasserlösliche und/oder fettlösliche aktive Bestandteil(e) (4) in der Klebmatrix (3) eingebettet aufweist.

10. Maske nach einem der vorstehenden Ansprüche, **dadurch gekennzeichnet, dass** die Klebmatrix (3) in einer Menge zwischen 15 und 70 g/m² auf dem Trägerbogen (2) abgeschieden ist.

11. Maske nach einem der vorstehenden Ansprüche, **dadurch gekennzeichnet, dass** das Adhesiv aus der folgenden Liste gewählt ist: Adhesive auf Acrylsäure-, Vinyl-, Polyurethan-, Silikon- oder Elastomerbasis, deren Copolymeren und/oder Mischungen derselben.

12. Maske nach einem der vorstehenden Ansprüche, **dadurch gekennzeichnet, dass** sie eine oder mehrere wasserabsorbierende Substanzen eingebettet in die Klebmatrix (3) aufweist, beispielsweise Polyacrylate oder Alginate, Derivate von Maisstärke oder Cellulose, natürliche oder synthetische wasserabsorbierende Teilchen oder Fasern.

13. Maske nach einem der vorstehenden Ansprüche, **dadurch gekennzeichnet, dass** der Trägerbogen (2) aus einem wasserquellbaren Material realisiert ist.

14. Maske nach einem der vorstehenden Ansprüche, **dadurch gekennzeichnet, dass** der Trägerbogen (2) aus einem elastischen Gewebe besteht.

15. Maske nach einem der vorstehenden Ansprüche, **dadurch gekennzeichnet, dass** der Trägerbogen aus einem hydrophilen nicht gewebten Gewebe besteht.

16. Maske nach einem der vorstehenden Ansprüche, **dadurch gekennzeichnet, dass** der Trägerbogen eine oder mehrere wasserabsorbierende Substanzen oder Teilchen oder Fasern trägt.

17. Maske nach einem der zwei vorstehenden Ansprüche, **dadurch gekennzeichnet, dass** das nicht gewebte Gewebe einen wesentlichen Anteil an Viskose oder Baumwolle aufweist, vorzugsweise zwischen 30 und 90 % (in Gewicht).

18. Maske nach einem der vorstehenden Ansprüche, **dadurch gekennzeichnet, dass** der Trägerbogen Polypropylen oder einen Polyester aufweist, vorzugsweise in einer Menge zwischen 5 und 95 % (in Gewicht).

19. Maske nach einem der vorstehenden Ansprüche, **dadurch gekennzeichnet, dass** das Adhesiv und der Trägerbogen so gewählt sind, dass die Maske dann, wenn sie einmal angefeuchtet ist, eine Oberfläche im gedehnten Zustand von mehr als 5 bis 70 % der Oberfläche der Maske im trockenen Zustand und vorzugsweise mehr als 10 bis 70 % und noch stärker bevorzugt oberhalb von 20 bis 70 % aufweist.

20. Maske nach einem der vorstehenden Ansprüche, **dadurch gekennzeichnet, dass** sie aktive Bestandteile (4) aufweist, ausgewählt zum Ausüben einer Wirkung, ausgewählt aus der folgenden Liste: hydratisierend, einebnend bzw. nivellierend, aufhellend, kräftigend bzw. belebend, verschlankend, heilend bzw. Narben bildend, betäubend.

21. Maske nach einem der vorstehenden Ansprüche, **dadurch gekennzeichnet, dass** der Trägerbogen nicht dehnbar ist, solange er nicht mit Wasser getränkt ist, und vorzugsweise auch nicht dehnbar ist, wenn er bei Herstellung der Maske in Kontakt mit der sich im fluiden Zustand, in gelöster Phase befindlichen Matrix tritt.

22. Verwendung einer Maske, wie sie in einem der vorstehenden Ansprüche definiert ist, zur Behandlung des Gesichts oder des Halses.

23. Verwendung einer Maske, wie sie in einem der vorstehenden Ansprüche 1 bis 21 definiert ist, zur Behandlung der Schenkel.

24. Verwendung einer Maske, wie sie in einem der Ansprüche 1 bis 21 definiert ist, wobei die vor der Anwendung in Wasser eingetauchte Maske sich nach Anwendung auf der vorzugsweise zuvor angefeuchteten Haut auf solche Weise gedehnt wird, dass sie sich an die Form bzw. Morphologie des Anwenders anpasst.

25. Verwendung einer Maske, wie sie in einem der Ansprüche 1 bis 21 definiert ist, **dadurch gekennzeichnet, dass** sie in trockenem Zustand auf angefeuchtete Haut angewandt wird und der Trägerbogen dann mit Wasser besprengt wird.

## Claims

1. A mask for the face or the body, which mask comprises a backing sheet coated on one of its faces with an adhesive matrix containing a permanent adhesive that adheres both to dry skin and to wet skin, at least one of the backing sheet and the adhesive matrix containing at least one active ingredient (4) serving to exert a specific action on the skin, said mask being
**characterized by** the fact that the adhesive matrix (3) is stretchable at least in the wet state, in order to enable the mask to be adapted to fit the shape of the face or of the portion of the body to be treated, the backing sheet (2) being chosen to be capable, at least in the wet state, of stretching so as to accompany the deformations in the adhesive matrix (3).

2. A mask according to claim 1, **characterized by** the fact that, during use, it has an elongation factor in at least one direction that lies in the range 5% to 30%, preferably in the range 10% to 30%, and more preferably in the range 15% to 30%.

3. A mask according to claim 1 or 2, **characterized by** the fact that the adhesive is hydrophobic.

4. A mask according to any preceding claim, **characterized by** the fact that the adhesive matrix (3) forms an occlusive layer.

5. A mask according to any preceding claim, **characterized by** the fact that the backing sheet (2) is provided with perforations (5; 6) and/or with cutouts (7; 8).

6. A mask according to the preceding claim, **characterized by** the fact that the backing sheet is provided with diamond-shaped perforations (6).

7. A mask according to claim 5, **characterized by** the fact that the backing sheet is provided with slots (7) disposed in staggered manner.

8. A mask according to any preceding claim, **characterized by** the fact that the adhesive matrix (3) is chosen such that, when it is applied in the runny state on the backing sheet during manufacture of the mask, it has viscosity preventing it from passing through said backing sheet.

9. A mask according to any preceding claim, **characterized by** the fact that it contains one or more active ingredients (4) that are water-soluble and/or liposoluble, and that are embedded in the adhesive matrix (3).

10. A mask according to any preceding claim, **characterized by** the fact that the adhesive matrix (3) is deposited in a quantity lying in the range 15 g/m² to 70 g/m² on the backing sheet (2).

11. A mask according to any preceding claim, **characterized by** the fact that the adhesive is chosen from the following list: adhesives based on acrylic, vinyl, polyurethane, silicone, or elastomer, and copolymers and/or mixtures thereof.

12. A mask according to any preceding claim, **characterized by** the fact that it contains one or more water-absorbant substances embedded in the adhesive matrix (3), e.g. polyacrylates or alginates, derivatives of corn or of cellulose, and natural or synthetic water-absorbant fibers or particles.

13. A mask according to any preceding claim, **characterized by** the fact that the backing sheet (2) is made of a material that swells in water.

14. A mask according to any preceding claim, **characterized by** the fact that the backing sheet (2) is constituted by an elastic woven fabric.

15. A mask according to any preceding claim, **characterized by** the fact that the backing sheet is constituted by a hydrophilic non-woven fabric.

16. A mask according to any preceding claim, **characterized by** the fact that the backing sheet contains one or more water-absorbant fibers or particles or substances.

17. A mask according to any preceding claim, **characterized by** the fact that the non-woven fabric contains a large proportion of viscose or of cotton, preferably in the range 30% by weight to 90% by weight.

18. A mask according to any preceding claim, **characterized by** the fact that the backing sheet contains polypropylene or polyester, preferably in a proportion (by weight) lying in the range 5% to 95%.

19. A mask according to any preceding claim, **characterized by** the fact that the adhesive and the backing sheet are chosen such that, once it is wet, the mask has a surface area in the stretched state that is greater by in the range 5% to 70% than the area of the mask in the dry state, and preferably greater by in the range 10% to 70%, and more preferably greater by in the range 20% to 70%.

20. A mask according to any preceding claim, **characterized by** the fact that it contains active ingredients (4) chosen to exert an action chosen from the following list: moistening, smoothing, lightening, toning up, slimming, healing, and anesthetizing.

21. A mask according to any preceding claim, **characterized by** the fact that the backing sheet is not stretchable so long as it is not impregnated with water, and preferably it is not stretchable when it is in contact with the matrix in the runny state, in solvent phase, during manufacture of the mask.

22. The use of a mask as defined in any preceding claim for treating the face or the neck.

23. The use of a mask as defined in any one of claims 1 to 21 for treating the thighs.

24. The use of a mask as defined in any one of claims 1 to 21, the mask being immersed in water before it is applied, and then being applied to the preferably pre-wetted skin by being stretched so as to be adapted to fit the morphology of the user.

25. The use of the mask as defined in any one of claims 1 to 21, **characterized by** the fact that said mask is applied in the dry state on wet skin, and then the backing sheet is sprayed with water.
